# FASCICULE DE BREVET EUROPEEN

(11) **EP 3 271 316 B1**
(45) Date de publication et mention de la délivrance du brevet: **22.04.2020**
(21) Numéro de dépôt: 16713552.4
(22) Date de dépôt: 15.03.2016
(51) Int. Cl.: C07C 11/10, C07C 11/107, C07C 21/18

(54) **STABILISATION DU 1-CHLORO-3,3,3-TRIFLUOROPROPENE**
STABILISIERUNG VON 1-CHLORO-3,3,3-TRIFLUORPROPEN
STABILISATION OF 1-CHLORO-3,3,3-TRIFLUOROPROPENE

(30) Priorité: 18.03.2015 FR 1552222
(43) Date de publication de la demande: 24.01.2018
(73) Titulaire: Arkema France, 92700 Colombes (FR)
(72) Inventeur: RACHED, Wissam, 69630 Chaponost (FR); GUERIN, Sophie, 69340 Francheville (FR); KINDLER, Pascale, 69270 Fontaines Sur Saone (FR)
(74) Mandataire: Dang, Doris
(86) Numéro de dépôt international: PCT/FR2016/050577
(87) Numéro de publication internationale: WO 2016/146940

(56) Documents cités:
- WO-A1-2009/003165
- WO-A1-2014/158663
- US-A1- 2013 004 435

## Description

### DOMAINE DE L'INVENTION

La présente invention concerne des composés permettant de stabiliser le 1-chloro-3,3,3-trifluoropropène et plus précisément de limiter ou d'empêcher l'isomérisation de la forme trans en forme cis. L'invention concerne également l'utilisation de tels stabilisants dans des applications de transfert de chaleur.

### ARRIERE-PLAN TECHNIQUE

Le trans-1-chloro-3,3,3-trifluoropropène (HCFO-1233zdE) est un produit présentant un faible potentiel de réchauffement climatique (GWP). Il possède des propriétés thermodynamiques et thermophysiques très favorables pour une utilisation comme fluide de transfert de chaleur dans les applications de refroidissement, de climatisation, de production d'électricité (notamment au moyen de cycles organiques de Rankine) et de pompes à chaleur à haute température.

Le HCFO-1233zdE présente une instabilité qui se manifeste surtout à température relativement élevée. Cette instabilité consiste en une isomérisation d'une fraction de la charge initiale aboutissant à la formation de cis-1-chloro-3,3,3-trifluoropropène (HCFO-1233zdZ).

Or, le HCFO-1233zdZ est un produit moins volatile que le HCFO-1233zdE. La température d'ébullition est de l'ordre de 40°C pour l'isomère Z, et de l'ordre de 18,3°C pour l'isomère E. Cette différence implique un changement des propriétés thermodynamiques et thermophysiques du produit dans les installations, et une perte de performance, lorsque l'isomérisation se produit.

Le document WO 2009/003165 décrit les risques de dégradation des hydrofluorooléfines et des hydrochlorofluorooléfines, ainsi que des stabilisants permettant de lutter contre cette dégradation. Ces stabilisants comprennent des composés piégeant les radicaux libres, des composés piégeant les acides, des composés piégeant l'oxygène et des inhibiteurs de polymérisation. Sont en particulier cités : le 1,2-époxybutane, le glycidyl méthyléther, l'oxyde de d-I-limonène, le 1,2-époxyméthylpropane, le nitrométhane, l'alpha méthylstyrène, l'isoprène, le phénol, les hydroquinones et l'hydrazine.

Le document US 7,795,480 décrit un procédé de fabrication du 2-chloro-3,3,3-trifluoropropène (HCFO-1233xf). Un phénomène de polymérisation du composé est mentionné (mais non un phénomène d'isomérisation). Des stabilisants proposés sont le p-tap(4-tert-amylphénol), la méthoxyhydroquinone, le 4-méthoxyphénol, la triéthylamine, la di-isopropylamine, l'hydroxyanisole butylée et le thymol.

Le document US 8,217,208 décrit le phénomène d'isomérisation du HFO-1233zdE sous l'effet de température, mais il n'enseigne pas de stabilisants permettant de limiter cette isomérisation.

Le document US 2012/0226081 décrit les risques de dégradation des hydrochlorooléfines et des hydrochloroalcanes, et propose un ensemble de stabilisants possibles : l'alpha-méthylstyrène, l'alpha-pinènoxyde, le beta-pinènoxyde, le 1,2-époxybutane, le 1,2-hexadécène-oxyde et les composés piégeant l'oxygène tels que la diéthylhydroxylamine, l'hydroquinone, la méthyléthylcétooxime et le p-méthoxyphénol.

Le document US 2015/0034523 décrit les risques de dégradation des hydrochlorooléfines et propose deux familles de stabilisants, à savoir les morpholines ou les trialkyl phosphates.

La quasi-totalité des stabilisants proposés dans l'état de la technique sont des produits solides, ou des produits liquides ayant une température d'ébullition élevée. Par exemple, la température d'ébullition de l'alpha-méthylstyrene est de 165°C, la température d'ébullition du limonène-oxyde est supérieure à 200°C, etc.

L'isoprène, mentionné dans le document WO 2009/003165, est quant à lui un produit instable en lui-même, qui doit généralement être combiné à un composé tel que le 4-tert-butylpyrocatéchol pour éviter sa polymérisation.

Le document WO 2014/158663 décrit des méthodes de purification et stabilisation des hydrofluorooléfines et hydrochlorofluorooléfines.

Le document US 2013/004435 divulgue une composition azéotropique de trans-1233zd et cyclopentène.

Les caractéristiques décrites ci-dessus rendent les stabilisants impropres à certaines applications dans lesquelles le HCFO-1233zdE est susceptible d'être employé. C'est en particulier le cas des applications employant des évaporateurs noyés (notamment avec des compresseurs sans huile de lubrification). Dans de telles applications, les stabilisants de l'état de la technique, à température d'ébullition élevée, sont inefficaces, car ils se concentrent dans l'évaporateur et ne migrent pas avec le fluide de transfert de chaleur au condenseur.

Il existe donc un besoin de fournir des stabilisants permettant de limiter ou d'empêcher l'isomérisation du HCFO-1233zdE en HCFO-1233zdZ, notamment dans des systèmes de compression de vapeur tels que des systèmes de climatisation, de réfrigération, de pompe à chaleur et de cycle organique de Rankine, et tout particulièrement les systèmes comportant un évaporateur noyé.

### RESUME DE L'INVENTION

L'invention concerne en premier lieu l'utilisation d'un composé alcène en C3 à C6 et comportant une seule double liaison, pour limiter ou empêcher l'isomérisation du trans-1-chloro-3,3,3-trifluoropropène en cis-1-chloro-3,3,3-trifluoropropène.

Selon un mode de réalisation, le composé alcène est un butène ou un pentène.

Selon un mode de réalisation, le composé alcène présente :
- une température d'ébullition inférieure ou égale à 100°C, de préférence inférieure ou égale à 75°C, et de manière plus particulièrement préférée inférieure ou égale à 50°C ; et/ou
- une température de solidification inférieure ou égale à 0°C, de préférence inférieure ou égale à -25°C, et de manière plus particulièrement préférée inférieure ou égale à -50°C.

Selon un mode de réalisation, le composé alcène est le 2-méthyl-but-2-ène.

Selon un mode de réalisation, le composé alcène est le 3-méthyl-but-1-ène.

L'invention a également pour objet une composition comprenant du 1-chloro-3,3,3-trifluoropropène et un composé alcène en C3 à C6 à chaîne linéaire ou ramifiée et comportant une seule double liaison.

Selon un mode de réalisation, le composé alcène est un butène ou un pentène.

Selon un mode de réalisation, le composé alcène présente :
- une température d'ébullition inférieure ou égale à 100°C, de préférence inférieure ou égale à 75°C, et de manière plus particulièrement préférée inférieure ou égale à 50°C ; et/ou
- une température de solidification inférieure ou égale à 0°C, de préférence inférieure ou égale à -25°C, et de manière plus particulièrement préférée inférieure ou égale à -50°C.

Selon un mode de réalisation, le composé alcène est le 2-méthyl-but-2-ène.

Selon un mode de réalisation, le composé alcène est le 3-méthyl-but-1-ène.

Selon un mode de réalisation, la composition comprend de 0,01 à 5 %, de préférence de 0,1 à 2 % et plus particulièrement de 0,2 à 1 %, en masse, de composé alcène.

Selon un mode de réalisation, le 1-chloro-3,3,3-trifluoropropène est sous forme trans dans une proportion massique supérieure ou égale à 90 %, de préférence supérieure ou égale à 95 %, de manière plus particulièrement préférée supérieure ou égale à 98 %, de manière encore plus particulièrement préférée supérieure ou égale à 99 %, et idéalement supérieure ou égale à 99,5 % voire supérieure à 99,9 %.

Selon un mode de réalisation, la composition comprend en outre un ou plusieurs composés de transfert de chaleur différents du 1-chloro-3,3,3-trifluoropropène et/ou un ou plusieurs additifs choisis parmi des stabilisants différents du composé alcène, des lubrifiants, des tensioactifs, des agents traceurs, des agents fluorescents, des agents odorants, des agents de solubilisation et leurs mélanges.

L'invention a également pour objet l'utilisation de la composition ci-dessus en tant que fluide de transfert de chaleur dans un système de compression de vapeur.

Selon un mode de réalisation, le système de compression de vapeur est :
- un système de climatisation ; ou
- un système de réfrigération ; ou
- un système de congélation ; ou
- un système de pompe à chaleur.

Selon un mode de réalisation, l'utilisation ci-dessus est une utilisation en tant que fluide de transfert de chaleur dans un moteur thermique.

Selon un mode de réalisation, le fluide de transfert de chaleur est à une température supérieure ou égale à 100°C, de préférence supérieure ou égale à 140°C, de manière plus particulièrement préférée supérieure ou égale à 180°C, pendant au moins une fraction de sa durée d'utilisation.

Selon un mode de réalisation, le fluide de transfert de chaleur est évaporé dans un évaporateur noyé.

L'invention a également pour objet une installation de transfert de chaleur comprenant un circuit contenant la composition ci-dessus en tant que fluide de transfert de chaleur.

Selon un mode de réalisation, l'installation est choisie parmi les installations mobiles ou stationnaires de chauffage par pompe à chaleur, de climatisation, de réfrigération, de congélation et les moteurs thermiques.

Selon un mode de réalisation, l'installation comprend un évaporateur noyé.

L'invention a également pour objet un procédé de chauffage ou de refroidissement d'un fluide ou d'un corps au moyen d'un système de compression de vapeur contenant un fluide de transfert de chaleur, ledit procédé comprenant successivement l'évaporation du fluide de transfert de chaleur, la compression du fluide de transfert de chaleur, la condensation du fluide de chaleur et la détente du fluide de transfert de chaleur, dans lequel le fluide de transfert de chaleur est la composition décrite ci-dessus.

L'invention a également pour objet un procédé de production d'électricité au moyen d'un moteur thermique, ledit procédé comprenant successivement l'évaporation du fluide de transfert de chaleur, la détente du fluide de transfert de chaleur dans une turbine permettant de générer de l'électricité, la condensation du fluide de chaleur et la compression du fluide de transfert de chaleur, dans lequel le fluide de transfert de chaleur est la composition décrite ci-dessus.

La présente invention permet de surmonter les inconvénients de l'état de la technique. Elle fournit plus particulièrement des stabilisants permettant de limiter ou d'empêcher l'isomérisation du HCFO-1233zdE en HCFO-1233zdZ, notamment dans des systèmes de compression de vapeur tels que des systèmes de climatisation, de réfrigération, de pompe à chaleur et de moteur thermique, et tout particulièrement les systèmes comportant un évaporateur noyé.

### DESCRIPTION DE MODES DE REALISATION DE L'INVENTION

L'invention est maintenant décrite plus en détail et de façon non limitative dans la description qui suit.

Sauf mention contraire, dans l'ensemble de la demande les proportions de composés indiquées sont données en pourcentages massiques.

L'invention repose sur la découverte que les composés alcènes en C3 à C6 comportant une seule double liaison permettent de stabiliser le HCFO-1233zdE, c'est-à-dire de limiter ou d'empêcher son isomérisation en HCFO-1233zdZ, notamment à des températures élevées.

Les composés stabilisants de l'invention sont donc le propène, les butènes, les pentènes et les hexènes. Les butènes et les pentènes sont préférés. Les pentènes sont encore plus particulièrement préférés.

Les composés stabilisants de l'invention peuvent être à chaîne linéaire ou ramifiée et de préférence ramifiée.

De préférence, ils présentent une température d'ébullition inférieure ou égale à 100°C, de préférence encore inférieure ou égale à 75°C, et de manière plus particulièrement préférée inférieure ou égale à 50°C.

Par « *température d'ébullition* », on entend la température d'ébullition à une pression de 101,325 kPa, telle que déterminée selon la norme NF EN 378-1 d'avril 2008.

De préférence également, ils présentent une température de solidification inférieure ou égale à 0°C, de préférence inférieure ou égale à -25°C, et de manière plus particulièrement préférée inférieure ou égale à -50°C.

La température de solidification est déterminée selon *l'Essai n° 102: Point de fusion*/*Intervalle de fusion* (Lignes directrices de l'OCDE pour les essais de produits chimiques, Section 1, Éditions OCDE, Paris, 1995, disponible à l'adresse http://dx.doi.org/10.1787/9789264069534-fr).

Des composés stabilisants de l'invention sont notamment :
- lebut-1-ène;
- le cis-but-2-ène ;
- le trans-but-2-ène ;
- le 2-méthylprop-1-ène ;
- le pent-1-ène ;
- le cis-pent-2-ène ;
- le trans-pent-2-ène ;
- le 2-méthylbut-1-ène ;
- le 2-méthylbut-2-ène ; et
- le 3-méthylbut-1-ène.

Parmi les composés préférés, on compte notamment le 2-méthyl-but-2-ène, de formule (CH3)₂C=CH-CH₃ (température d'ébullition de 39°C environ) ; et le 3-méthyl-but-1-ène, de formule CH₃-CH(CH₃)-CH=CH₂ (température d'ébullition de 25°C environ).

Deux ou plus de deux des composés ci-dessus peuvent également être utilisés en combinaison.

Les composés stabilisants selon l'invention sont ainsi avantageusement utilisés en association avec du HCFO-1233zd, et plus particulièrement avec du HCFO-1233zdE, dans des applications de transfert de chaleur.

Ainsi, l'invention propose une composition, notamment utile pour les applications de transfert de chaleur, comprenant au moins du HCFO-1233zd et un composé stabilisant décrit ci-dessus.

La proportion massique des composés stabilisants ci-dessus dans la composition peut notamment être : de 0,01 à 0,05 % ; ou de 0,05 à 0,1 % ; ou de 0,1 à 0,2 %, ou de 0,2 à 0,3 % ; ou de 0,3 à 0,4 % ; ou de 0,4 à 0,5 % ; ou de 0,5 à 0,6 % ; ou de 0,6 à 0,7 % ; ou de 0,7 à 0,8 % ; ou de 0,8 à 0,9 % ; ou de 0,9 à 1 % ; ou de 1 à 1,2 % ; ou de 1,2 à 1,5 %, ou de 1,5 à 2 % ; ou de 2 à 3 % ; ou de 3 à 4 % ; ou de 4 à 5 %.

La composition peut comprendre du HCFO-1233zdE et éventuellement du HCFO-1233zdZ. Avantageusement, la proportion de HCFO-1233zdE, par rapport au total du HCFO-1233zd, est supérieure ou égale à 90 %, ou à 91 %, ou à 92 %, ou à 93 %, ou à 94 %, ou à 95 %, ou à 96 %, ou à 97 %, ou à 98 %, ou à 99 %, ou à 99,1 %, ou à 99,2 %, ou à 99,3 %, ou à 99,4 %, ou à 99,5 %, ou à 99,6 %, ou à 99,7 %, ou à 99,8 %, ou à 99,9 %, ou à 99,91 %, ou à 99,92 %, ou à 99,93 %, ou à 99,94 %, ou à 99,95 %, ou à 99,96 %, ou à 99,97 %, ou à 99,98 %, ou à 99,99 %.

La présence du composé stabilisant permet de limiter ou d'empêcher une augmentation de la proportion de HCFO-1233zdZ dans la composition au cours du temps et / ou en cas d'application de températures relativement élevées.

La composition de l'invention peut également comporter des additifs divers. Dans le cas où il s'agit d'une composition de transfert de chaleur, les additifs peuvent notamment être choisis parmi les lubrifiants, les nanoparticules, les stabilisants (différents des composés stabilisants de l'invention), les tensioactifs, les agents traceurs, les agents fluorescents, les agents odorants et les agents de solubilisation.

Le ou les stabilisants, lorsqu'ils sont présents, représentent de préférence au plus 5 % en masse dans la composition de transfert de chaleur. Parmi les stabilisants, on peut citer notamment le nitrométhane, l'acide ascorbique, l'acide téréphtalique, les azoles tels que le tolutriazole ou le benzotriazole, les composés phénoliques tels que le tocophérol, l'hydroquinone, le t-butyl hydroquinone, le 2,6-di-ter-butyl-4-méthylphénol, les époxydes (alkyl éventuellement fluoré ou perfluoré ou alkényl ou aromatique) tels que les n-butyl glycidyl éther, hexanediol diglycidyl éther, allyl glycidyl éther, butylphénylglycidyl éther, les phosphites, les phosphonates, les thiols et les lactones.

A titre de lubrifiants on peut notamment utiliser des huiles d'origine minérale, des huiles de silicone, des paraffines d'origine naturelle, des naphtènes, des paraffines synthétiques, des alkylbenzènes, des poly-alpha oléfines, des polyalkène glycols, des polyol esters et / ou des polyvinyléthers.

Selon un mode de réalisation avantageux de l'invention, la composition de l'invention est toutefois dépourvue de lubrifiant.

A titre de nanoparticules on peut notamment utiliser les nanoparticules de charbon, les oxydes métalliques (cuivre, aluminium), TiO₂, Al₂O₃, MoS₂...

A titre d'agents traceurs (susceptibles d'être détectés) on peut citer les hydrofluorocarbures deutérés ou non, les hydrocarbures deutérés, les perfluorocarbures, les fluoroéthers, les composés bromés, les composés iodés, les alcools, les aldéhydes, les cétones, le protoxyde d'azote et les combinaisons de ceux-ci. L'agent traceur est différent du ou des composés de transfert de chaleur composant le fluide de transfert de chaleur.

A titre d'agents de solubilisation, on peut citer les hydrocarbures, le diméthyléther, les polyoxyalkylène éthers, les amides, les cétones, les nitriles, les chlorocarbures, les esters, les lactones, les aryl éthers, les fluoroéthers et les 1,1,1-trifluoroalcanes. L'agent de solubilisation est différent du ou des composés de transfert de chaleur composant le fluide de transfert de chaleur.

A titre d'agents fluorescents, on peut citer les naphthalimides, les perylènes, les coumarines, les anthracènes, les phénanthracènes, les xanthènes, les thioxanthènes, les naphthoxanhtènes, les fluorescéines et les dérivés et combinaisons de ceux-ci.

A titre d'agents odorants, on peut citer les alkylacrylates, les allylacrylates, les acides acryliques, les acrylesters, les alkyléthers, les alkylesters, les alcynes, les aldéhydes, les thiols, les thioéthers, les disulfures, les allylisothiocyanates, les acides alcanoïques, les aminés, les norbornènes, les dérivés de norbornènes, le cyclohexène, les composés aromatiques hétérocycliques, l'ascaridole, l'o-méthoxy(méthyl)-phénol et les combinaisons de ceux-ci.

La composition selon l'invention peut également comprendre au moins un autre composé de transfert de chaleur, en plus du HCFO-1233zd. Un tel autre composé de transfert de chaleur optionnel peut être notamment un composé hydrocarbure, éther, hydrofluoroéther, hydrofluorocarbure, hydrochlorofluorocarbure, hydrofluorooléfine, hydrochlorooléfine ou hydrochlorofluorooléfine.

A titre d'exemple, ledit autre composé de transfert de chaleur peut être choisi parmi le 1,1,1,4,4,4-hexafluorobut-2-ène (HFO-1336mmz, isomère E ou Z), le 3,3,4,4,4-pentafluorobut-1-ène (HFO-1345fz), le 2,4,4,4-tétrafluorobut-1-ène (HFO-1354mfy), le 1,1,1,3,3-pentafluoropropane (HFC-245fa), le 2,3,3,3-tétrafluoropropène (HFO-1234yf), le 1,3,3,3-tétrafluoropropène (HFO-1234ze), le difluorométhane (HFC-32), le 1,1,1,2-tétrafluoroéthane (HFC-134a), le 1,1,2,2-tétrafluoroéthane (HFC-134), le 1,1-difluoroéthane (HFC-152a), le pentafluoroéthane (HFC-125), le 1,1,1,3,3-pentafluorobutane (HFC-365mfc), le méthoxynonafluorobutane (HFE7100), le butane (HC-600), le 2-méthylbutane (HC-601a), le pentane (HC-601), l'éthyl éther, le méthyl acétate et les combinaisons de ceux-ci.

Dans la composition de l'invention, le HCFO-1233zd peut représenter notamment de 1 à 5 % de la composition ; ou de 5 à 10 % de la composition ; ou de 10 à 15 % de la composition ; ou de 15 à 20 % de la composition ; ou de 20 à 25 % de la composition ; ou de 25 à 30 % de la composition ; ou de 30 à 35 % de la composition ; ou de 35 à 40 % de la composition ; ou de 40 à 45 % de la composition ; ou de 45 à 50 % de la composition ; ou de 50 à 55 % de la composition ; ou de 55 à 60 % de la composition ; ou de 60 à 65 % de la composition ; ou de 65 à 70 % de la composition ; ou de 70 à 75 % de la composition ; ou de 75 à 80 % de la composition ; ou de 80 à 85 % de la composition ; ou de 85 à 90 % de la composition ; ou de 90 à 95 % de la composition ; ou de 95 à 99 % de la composition ; ou de 99 à 99,5 % de la composition ; ou de 99,5 à 99,9 % de la composition ; ou plus de 99,9 % de la composition. La teneur en HCFO-1233zd peut également varier dans plusieurs des intervalles ci-dessus : par exemple de 50 à 55 % et de 55 à 60 %, c'est-à-dire de 50 à 60 %, etc.

La composition de l'invention peut être utilisée dans un procédé de transfert de chaleur.

Le procédé de transfert de chaleur selon l'invention repose sur l'utilisation d'une installation comprenant un système de compression de vapeur qui contient la composition de l'invention en tant que fluide de transfert de chaleur. Le procédé de transfert de chaleur peut être un procédé de chauffage ou de refroidissement d'un fluide ou d'un corps.

La composition de l'invention peut aussi être utilisée dans un procédé de production de travail mécanique ou d'électricité, notamment conformément à un cycle de Rankine.

Pour les applications de chauffage et de refroidissement, le système de compression de vapeur comprend au moins un évaporateur, un compresseur, un condenseur et un détendeur, ainsi que des lignes de transport de fluide de transfert de chaleur entre ces éléments. L'évaporateur et le condenseur comprennent un échangeur de chaleur permettant un échange de chaleur entre le fluide de transfert de chaleur et un autre fluide ou corps.

A titre de compresseur, on peut utiliser notamment un compresseur centrifuge à un ou plusieurs étages ou un mini-compresseur centrifuge. Les compresseurs rotatifs, spirales, à piston ou à vis peuvent aussi être utilisés. Le compresseur peut être entraîné par un moteur électrique ou par une turbine à gaz (par exemple alimentée par les gaz d'échappement d'un véhicule, pour les applications mobiles) ou par engrenage.

Le système de compression de vapeur fonctionne alors selon un cycle classique de compression de vapeur. Le cycle comprend le changement d'état du fluide de transfert de chaleur d'une phase liquide (ou diphasique liquide / vapeur) vers une phase vapeur à une pression relativement faible, puis la compression du fluide en phase vapeur jusqu'à une pression relativement élevée, le changement d'état (condensation) du fluide de transfert de chaleur de la phase vapeur vers la phase liquide à une pression relativement élevée, et la réduction de la pression pour recommencer le cycle.

L'installation peut également éventuellement comprendre au moins un circuit de fluide caloporteur utilisé pour transmettre la chaleur (avec ou sans changement d'état) entre le circuit de fluide de transfert de chaleur et le fluide ou corps à chauffer ou refroidir.

L'installation peut également éventuellement comprendre deux systèmes de compression de vapeur (ou plus), contenant des fluides de transfert de chaleur identiques ou distincts. Par exemple, les systèmes de compression de vapeur peuvent être couplés entre eux.

Les procédés et installations de refroidissement selon l'invention comprennent les procédés et installations de climatisation (avec des installations mobiles, par exemple dans des véhicules, ou stationnaires), de réfrigération (avec des installations mobiles par exemple dans les containers, ou stationnaires) et de congélation ou de cryogénie.

Les installations de chauffage selon l'invention comprennent les pompes à chaleur.

Pour les applications de production de travail mécanique ou d'électricité, l'installation est un moteur thermique, qui comprend au moins un évaporateur, une turbine, un condenseur et une pompe, ainsi que des lignes de transport de fluide de transfert de chaleur entre ces éléments. L'installation peut alors fonctionner selon un cycle de Rankine.

Il est possible d'utiliser tout type d'échangeur de chaleur pour la mise en œuvre des fluides de transfert de chaleur selon l'invention, et notamment des échangeurs de chaleur à co-courant ou, de préférence, des échangeurs de chaleur à contre-courant.

En particulier, l'évaporateur utilisé dans le cadre de l'invention peut être un évaporateur à surchauffe ou un évaporateur noyé. Dans un évaporateur à surchauffe, la totalité du fluide de transfert de chaleur est évaporé à la sortie de l'évaporateur, et la phase vapeur est surchauffée.

Dans un évaporateur noyé, le fluide de transfert de chaleur sous forme liquide ne s'évapore pas complètement. Un évaporateur noyé comporte un séparateur de phase liquide et de phase vapeur.

L'invention est particulièrement utile lorsqu'un tel évaporateur est utilisé. En effet, les stabilisants de l'état de la technique à température d'ébullition élevée sont inefficaces lorsqu'un tel évaporateur est employé, car ils se concentrent dans l'évaporateur et ne migrent pas avec le fluide de transfert de chaleur au condenseur.

L'invention est également particulièrement utile lorsqu'une température élevée existe en au moins un point du circuit de fluide, et plus particulièrement une température supérieure ou égale à 100°C, ou à 110°C, ou à 120°C, ou à 130°C, ou à 140°C, ou à 150°C, ou à 160°C, ou à 170°C, ou à 180°C, ou à 190°C, ou à 200°C. En effet, ce sont dans ces conditions que le HCFO-1233zdE est le plus susceptible de se convertir en HCFO-1233zdZ.

En particulier, dans les appareils de climatisation, la température générale de fonctionnement est inférieure à 100°C ; mais des points chauds à la sortie du compresseur peuvent atteindre des températures supérieures à 100°C, affectant le fluide de transfert de chaleur sur une faible proportion de sa durée de circulation complète (par exemple moins de 1 %).

Dans les pompes à chaleur, la température de condensation peut atteindre 140°C environ. Dans ce cas, le fluide de transfert de chaleur peut être à une température de 140°C environ sur une proportion importante de sa durée de circulation complète (par exemple environ 50 %). De plus, des points chauds entre 150 et 200°C peuvent également être constatés à la sortie du compresseur. L'impact d'une durée de séjour longue à des températures supérieures à 100°C et l'existence de points à des températures pouvant avoisiner les 200°C nécessitent donc un stabilisant.

De préférence également, dans l'installation selon l'invention, la température de la composition utilisée en tant que fluide de transfert de chaleur reste supérieure à la température de solidification du composé stabilisant, afin d'éviter tout dépôt de matière solide dans le circuit.

La composition selon l'invention peut également être utile en tant qu'agent d'expansion, agent de propulsion (par exemple pour un aérosol), agent de nettoyage ou solvant, gaz diélectrique, outre son utilisation en tant que fluide de transfert de chaleur.

En tant qu'agent de propulsion, la composition selon l'invention peut être utilisée seule ou en combinaison avec des agents de propulsion connus. L'agent de propulsion comprend, de préférence consiste en, une composition selon l'invention. La substance active devant être projetée peut être mélangée avec l'agent de propulsion et des composés inertes, des solvants ou autres additifs, pour former une composition à projeter. De préférence, la composition à projeter est un aérosol.

En tant qu'agent d'expansion, la composition selon l'invention peut être comprise dans une composition d'expansion, qui comprend de préférence un ou plusieurs autres composés susceptibles de réagir et de former une mousse ou structure cellulaire dans des conditions appropriées, comme cela est connu de l'homme du métier.

En particulier, l'invention propose un procédé de préparation d'un produit thermoplastique expansé comprenant d'abord la préparation d'une composition polymérique d'expansion. Typiquement, la composition polymérique d'expansion est préparée en plastifiant une résine polymère et en mélangeant les composés d'une composition d'agent d'expansion à une pression initiale. La plastification de la résine polymère peut être effectuée sous l'effet de la chaleur, en chauffant la résine polymère pour la ramollir suffisamment pour mélanger une composition d'agent d'expansion. Généralement, la température de plastification est proche de la température de transition vitreuse ou de la température de fusion pour les polymères cristallins.

D'autres utilisations de la composition selon l'invention comprennent les utilisations en tant que solvant, agent de nettoyage ou autres. On peut citer par exemple le dégraissage par la vapeur, le nettoyage de précision, le nettoyage de circuits électroniques, le nettoyage à sec, le nettoyage abrasif, les solvants pour le dépôt de lubrifiants et d'agents de libération, et d'autres traitements de solvant ou de surface.

### EXEMPLES

Les exemples suivants illustrent l'invention sans la limiter.

### Exemple 1 (comparatif) - instabilité du HCFO-1233zdE en l'absence de stabilisant

Les essais de stabilité thermique du HCFO-1233zdE sont réalisés suivant la norme ASHRAE 97-2007 intitulée « *Sealed glass tube method to test the chemical stability of materials for use within refrigerant systems ».*

Les compositions sont déterminées par chromatographe en phase gaz sur une colonne CP-sil8-CB.

Une première série d'essais est réalisée à 150°C pendant des durées comprises entre 10 minutes et 14 jours. Les résultats montrent une légère formation de l'isomère HFO-1233zdZ, jusqu'à atteindre une teneur de 0,14 % à 14 jours.

Une deuxième série d'essais est réalisée à 200°C pour une durée de 24 heures. Les résultats montrent une légère formation de l'isomère HCFO-1233zdZ jusqu'à atteindre 1 % environ.

Enfin, une troisième série d'essais est réalisée à 250°C pendant une durée de 24 heures également. Les résultats montrent une formation de l'isomère HCFO-1233zdZ comprise entre 6 et 9 %.

### Exemple 2 (invention) - stabilisation du HCFO-1233zdE

Des essais de stabilité thermique similaires à ceux de l'exemple 1 sont effectués, en ajoutant 0,5 % de stabilisant au HCFO-1233zdE (teneur massique par rapport à la somme du stabilisant et du HCFO-1233zdE). Les stabilisants testés sont le 2-méthyl-but-2-ène (2m2b) et le 3-méthyl-but-1-ène (3m1b).

Une première série d'essais est réalisée à 150°C pour une durée de 14 jours. Les essais avec le 3m1b montrent une formation de l'isomère HCFO-1233zdZ de l'ordre de 0,08 % à la fin de la période. Dans les essais avec le 2m2b, aucune formation de HFO-1233zd-Z n'est mesurée.

Une deuxième série d'essais est réalisée à 200°C pour une durée de 24 heures. Les essais avec le 3m1b montrent une légère formation de l'isomère HFO-1233zdZ de l'ordre de 0,3 %, et ceux avec le 2m2b montrent une formation de HCFO-1233zdZ de l'ordre de 0,07 % à la fin de cette période.

Le tableau suivant résume l'effet de stabilisation observé :

| | **HCFO-1233zdE seul** | **HCFO-1233zdE + 3m1b** | **HCFO-1233zdE + 2m2b** |
|---|---|---|---|
| **14 jours à 150°C** | 0,14 % de HCFO-1233zdZ | 0,08 % de HCFO-1233zdZ | HCFO-1233zdZ indétectable |
| **24 heures à 200°C** | 1 % de HCFO-1233zdZ | 0,3 % de HCFO-1233zdZ | 0,07 % de HCFO-1233zdZ |

## Revendications

1. Utilisation d'un composé alcène en C3 à C6 et comportant une seule double liaison, pour limiter ou empêcher l'isomérisation du trans-1-chloro-3,3,3-trifluoropropène en cis-1-chloro-3,3,3-trifluoropropène.

2. Utilisation selon la revendication 1, dans laquelle le composé alcène est un butène ou un pentène.

3. Utilisation selon la revendication 1 ou 2, dans laquelle le composé alcène présente :
- une température d'ébullition inférieure ou égale à 100°C, de préférence inférieure ou égale à 75°C, et de manière plus particulièrement préférée inférieure ou égale à 50°C ; et/ou
- une température de solidification inférieure ou égale à 0°C, de préférence inférieure ou égale à -25°C, et de manière plus particulièrement préférée inférieure ou égale à -50°C.

4. Utilisation selon la revendication 1, dans laquelle le composé alcène est le 2-méthyl-but-2-ène, ou le 3-méthyl-but-1-ène.

5. Composition comprenant du 1-chloro-3,3,3-trifluoropropène et un composé alcène en C3 à C6 à chaîne linéaire ou ramifiée et comportant une seule double liaison.

6. Composition selon la revendication 5, dans laquelle le composé alcène est un butène ou un pentène.

7. Composition selon la revendication 5 ou 6, dans laquelle le composé alcène présente :
- une température d'ébullition inférieure ou égale à 100°C, de préférence inférieure ou égale à 75°C, et de manière plus particulièrement préférée inférieure ou égale à 50°C ; et/ou
- une température de solidification inférieure ou égale à 0°C, de préférence inférieure ou égale à -25°C, et de manière plus particulièrement préférée inférieure ou égale à -50°C.

8. Composition selon la revendication 5, dans laquelle le composé alcène est le 2-méthyl-but-2-ène ou le 3-méthyl-but-1-ène.

9. Composition selon l'une des revendications 5 à 8, comprenant de 0,01 à 5 %, de préférence de 0,1 à 2 % et plus particulièrement de 0,2 à 1 %, en masse, de composé alcène.

10. Composition selon l'une des revendications 5 à 9, dans laquelle le 1-chloro-3,3,3-trifluoropropène est sous forme trans dans une proportion massique supérieure ou égale à 90 %, de préférence supérieure ou égale à 95 %, de manière plus particulièrement préférée supérieure ou égale à 98 %, de manière encore plus particulièrement préférée supérieure ou égale à 99 %, et idéalement supérieure ou égale à 99,5 % voire supérieure à 99,9 %.

11. Composition selon l'une des revendications 5 à 10, comprenant en outre un ou plusieurs composés de transfert de chaleur différents du 1-chloro-3,3,3-trifluoropropène et/ou un ou plusieurs additifs choisis parmi des stabilisants différents du composé alcène, des lubrifiants, des tensioactifs, des agents traceurs, des agents fluorescents, des agents odorants, des agents de solubilisation et leurs mélanges.

12. Utilisation d'une composition selon l'une des revendications 5 à 11 en tant que fluide de transfert de chaleur dans un système de compression de vapeur, de préférence :
- un système de climatisation ; ou
- un système de réfrigération ; ou
- un système de congélation ; ou
- un système de pompe à chaleur.

13. Utilisation d'une composition selon l'une des revendications 5 à 11 en tant que fluide de transfert de chaleur dans un moteur thermique.

14. Utilisation selon l'une des revendications 12 à 13, dans laquelle le fluide de transfert de chaleur est à une température supérieure ou égale à 100°C, de préférence supérieure ou égale à 140°C, de manière plus particulièrement préférée supérieure ou égale à 180°C, pendant au moins une fraction de sa durée d'utilisation.

15. Utilisation selon l'une des revendications 12 à 14, dans laquelle le fluide de transfert de chaleur est évaporé dans un évaporateur noyé.

## Patentansprüche

1. Verwendung einer C₃- bis C₆-Alkenverbindung mit einer einzigen Doppelbindung zur Einschränkung oder Verhinderung der Isomerisierung von trans-1-Chlor-3,3,3-trifluorpropen zu cis-1-Chlor-3,3,3-trifluorpropen.

2. Verwendung nach Anspruch 1, wobei es sich bei der Alkenverbindung um ein Buten oder ein Penten handelt.

3. Verwendung nach Anspruch 1 oder 2, wobei die Alkenverbindung
- einen Siedepunkt kleiner oder gleich 100 °C, vorzugsweise kleiner oder gleich 75 °C und spezieller bevorzugt kleiner oder gleich 50 °C und/oder
- eine Verfestigungstemperatur kleiner oder gleich 0 °C, vorzugsweise kleiner oder gleich -25 °C und spezieller bevorzugt kleiner oder gleich -50 °C
aufweist.

4. Verwendung nach Anspruch 1, wobei es sich bei der Alkenverbindung um 2-Methylbut-2-en oder 3-Methylbut-1-en handelt.

5. Zusammensetzung, umfassend 1-Chlor-3,3,3-trifluor-propen und eine C₃- bis C₆-Alkenverbindung mit linearer oder verzweigter Kette und einer einzigen Doppelbindung.

6. Zusammensetzung nach Anspruch 5, wobei es sich bei der Alkenverbindung um ein Buten oder ein Penten handelt.

7. Zusammensetzung nach Anspruch 5 oder 6, wobei die Alkenverbindung
- einen Siedepunkt kleiner oder gleich 100 °C, vorzugsweise kleiner oder gleich 75 °C und spezieller bevorzugt kleiner oder gleich 50 °C und/oder
- eine Verfestigungstemperatur kleiner oder gleich 0 °C, vorzugsweise kleiner oder gleich -25 °C und spezieller bevorzugt kleiner oder gleich -50 °C
aufweist.

8. Zusammensetzung nach Anspruch 5, wobei es sich bei der Alkenverbindung um 2-Methylbut-2-en oder 3-Methylbut-1-en handelt.

9. Zusammensetzung nach einem der Ansprüche 5 bis 8, umfassend 0,01 bis 5 Massen-%, vorzugsweise 0,1 bis 2 Massen-% und spezieller 0,2 bis 1 Massen-% Alkenverbindung.

10. Zusammensetzung nach einem der Ansprüche 5 bis 9, wobei das 1-Chlor-3,3,3-trifluorpropen in transForm in einem Massenanteil größer oder gleich 90 %, vorzugsweise größer oder gleich 95 %, spezieller bevorzugt größer oder gleich 98 %, noch spezieller bevorzugt größer oder gleich 99 % und idealerweise größer oder gleich 99,5 % oder sogar größer als 99,9 % vorliegt.

11. Zusammensetzung nach einem der Ansprüche 5 bis 10, umfassend außerdem eine oder mehrere Wärmeübertragungsverbindungen, die von 1-Chlor-3,3,3-trifluorpropen verschieden sind, und/oder ein oder mehrere Additive, die aus Stabilisatoren, die von der Alkenverbindung verschieden sind, Schmiermitteln, Tensiden, Tracern, Fluoreszenzmitteln, Geruchsmitteln, Solubilisatoren und Mischungen davon ausgewählt sind.

12. Verwendung einer Zusammensetzung nach einem der Ansprüche 5 bis 11 als Wärmeübertragungsfluid in einem Dampfverdichtungssystem, vorzugsweise
- einem Klimatisierungssystem oder
- einem Kälteerzeugungssystem oder
- einem Gefriersystem oder
- einem Wärmepumpensystem.

13. Verwendung einer Zusammensetzung nach einem der Ansprüche 5 bis 11 als Wärmeübertragungsfluid in einer Wärmekraftmaschine.

14. Verwendung nach einem der Ansprüche 12 bis 13, wobei das Wärmeübertragungsfluid während mindestens eines Teils seiner Verwendungsdauer bei einer Temperatur größer oder gleich 100 °C, vorzugsweise größer oder gleich 140 °C, spezieller bevorzugt größer oder gleich 180 °C vorliegt.

15. Verwendung nach einem der Ansprüche 12 bis 14, wobei das Wärmeübertragungsfluid in einem Überflutungsverdampfer verdampft wird.

## Claims

1. Use of a C₃ to C₆ alkene compound comprising a sole double bond, for limiting or preventing the isomerization of trans-1-chloro-3,3,3-trifluoropropene to cis-1-chloro-3,3,3-trifluoropropene.

2. Use according to Claim 1, wherein the alkene compound is a butene or a pentene.

3. Use according to Claim 1 or 2, wherein the alkene compound has:
- a boiling point less than or equal to 100°C, preferably less than or equal to 75°C, and more particularly preferably less than or equal to 50°C; and/or
- a solidification temperature less than or equal to 0°C, preferably less than or equal to -25°C, and more particularly preferably less than or equal to -50°C.

4. Use according to Claim 1, wherein the alkene compound is 2-methylbut-2-ene or 3-methylbut-1-ene.

5. Composition comprising 1-chloro-3,3,3-trifluoropropene and a C₃ to C₆ alkene compound with a linear or branched chain and comprising a sole double bond.

6. Composition according to Claim 5, wherein the alkene compound is a butene or a pentene.

7. Composition according to Claim 5 or 6, wherein the alkene compound has:
- a boiling point less than or equal to 100°C, preferably less than or equal to 75°C, and more particularly preferably less than or equal to 50°C; and/or
- a solidification temperature less than or equal to 0°C, preferably less than or equal to -25°C, and more particularly preferably less than or equal to -50°C.

8. Composition according to Claim 5, wherein the alkene compound is 2-methylbut-2-ene or 3-methylbut-1-ene.

9. Composition according to one of Claims 5 to 8, comprising from 0.01% to 5%, preferably from 0.1% to 2% and more particularly from 0.2% to 1%, by weight, of alkene compound.

10. Composition according to one of Claims 5 to 9, wherein the 1-chloro-3,3,3-trifluoropropene is in trans form in a weight proportion greater than or equal to 90%, preferably greater than or equal to 95%, more particularly preferably greater than or equal to 98%, even more particularly preferably greater than or equal to 99%, and ideally greater than or equal to 99.5%, or even greater than 99.9%.

11. Composition according to one of Claims 5 to 10, also comprising one or more heat-transfer compounds other than 1-chloro-3,3,3-trifluoropropene and/or one or more additives chosen from stabilizers other than the alkene compound, lubricants, surfactants, tracers, fluorescent agents, odorous agents, solubilizing agents, and mixtures thereof.

12. Use of a composition according to one of Claims 5 to 11 as a heat-transfer fluid in a vapor compression system, preferably:
- an air-conditioning system; or
- a refrigeration system; or
- a freezing system; or
- a heat pump system.

13. Use of a composition according to one of Claims 5 to 11 as a heat-transfer fluid in a thermal engine.

14. Use according to either of Claims 12 and 13, wherein the heat-transfer fluid is at a temperature greater than or equal to 100°C, preferably greater than or equal to 140°C, more particularly preferably greater than or equal to 180°C, for at least one fraction of the duration of its use.

15. Use according to one of Claims 12 to 14, wherein the heat-transfer fluid is evaporated in a flooded evaporator.
